# EUROPEAN PATENT APPLICATION

(11) **EP 3 760 714 A1**
(43) Date of publication of application: **06.01.2021**
(21) Application number: 19183869.7
(22) Date of filing: 02.07.2019
(51) Int. Cl.: C12N 9/24, C11D 3/386, A23K 20/189

(54) **IMPROVED MANNANASE VARIANTS**

(71) Applicant: AB Enzymes Oy, 05200 Rajamäki (FI)
(72) Inventor: JUNTUNEN, Kari, 05200 Rajamäki (FI); VALTAKARI, Leena, 05200 Rajamäki (FI); MÄKINEN, Susanna, 05200 Rajamäki (FI); OJA, Merja, 05200 Rajamäki (FI); HELLMUTH, Hendrik, 64293 Darmstadt (DE); OJAPALO, Pentti, 05200 Rajamäki (FI); LANGFELDER, Kim, 64293 Darmstadt (DE); PURANEN, Terhi, 05200 Rajamäki (FI)
(74) Representative: Espatent Oy

(57) **Abstract**

A variant of mannanase is disclosed having at least 90% sequence identity with SEQ ID NO: 1 and a substitution of an amino acid in position 256. An enzyme composition, detergent composition, host cell, animal feed, and feed supplement comprising the present variant are disclosed, as well as methods and uses involving the present variant.

## Description

### Field of the invention

The present invention is directed to the field of enzyme technology. In particular, disclosed herein are mannanase variants that have good mannan degrading performance and stability in different chemical environments, making them useful in various applications where degradation of mannan is desired, such as in laundry and cleaning applications, in feed and food technology, as well as in pulping industry, paper industry and oil industry.

### Background

Mannans are mannose containing polysaccharides found in various plants. Mannans are poorly soluble in an aqueous environment and their physicochemical properties give rise to viscous dispersions. Additionally, mannans have high water binding capacity. Each of these characteristics of mannan causes problems in industries where mannan containing materials are processed, such as in brewing, baking, animal nutrition, and in laundry and cleaning applications.

In plant-based diets different β-mannans are present and depending on their amounts and properties they can compromise nutrient digestion, microbial colonisation and growth. Enzymatic degradation of mannans can be used to reduce digesta viscosity. Degradation of mannans by mannanase enzymes results into production of manno-oligosaccharides. Use of mannanases in feeds increases average daily gain, feed efficiency, weight uniformity and livability in monogastric animals. For animal feed applications, such as in feeds for monogastric animals with cereal diets, mannan is a contributing factor to viscosity of gut contents and it thereby adversely affects the feed digestibility and animal growth rate. For ruminants, mannan represents a substantial component of fiber intake and a more complete digestion of mannan would facilitate higher feed conversion efficiency.

For laundry and cleaning applications, enzyme compositions comprising mannanase can be used to degrade mannan for example in mannan stains.

However, providing mannanases that are stable in varying storage and use conditions while still showing good mannan degrading activity is difficult. Thus, use of wild type mannanases in industrial applications is not always possible.

It is an object of the present invention to provide variants of mannanase that exhibit mannanase activity and that have performance and/or stability that allows their use in industrial processes. It is another object to provide variants that can be used in enzyme compositions for mannan degradation or modification.

### Summary of the invention

The present application concerns the inventions defined in the appended independent claims, and their embodiments disclosed below. The invention is based on development of mannanase variants by modifying a parent mannanase by protein engineering. The inventors have surprisingly found, and show in the examples below, that by substituting the amino acid position 256 of the present parent mannanase it is possible to obtain a variant which has improved, i.e. increased, performance in mannan degradation. The performance is improved particularly when used in detergents to wash stains containing mannan. Further, the stability of the variant can be improved by making additional substitutions in selected positions. The novel variants have good performance in wide range of detergents even with very low dosing.

According to the first aspect is provided a variant of mannanase, wherein the variant has:
at least 90%, but less than 100%, sequence identity with SEQ ID NO: 1,
a substitution of an amino acid in position 256, and
mannanase activity.

The present variant is advantageous in having good performance in degrading mannan. As shown in the examples provided below, the claimed variant has a performance, which is improved when compared to native, i.e. wild type mannanase to which the substitution is made. Thus, the claimed substitution position can be used alone, or with additional substitution positions, to improve the performance of a "parent" mannanase.

According to the second aspect is provided an enzyme composition comprising the variant of the first aspect, and:
a. at least one stabilizer selected from polyol, propylene glycol, polyethylene glycol, hexylene glycol, glycerol, a sugar, sugar alcohol, polysaccharide, lactic acid, peptide, surfactant, or a combination thereof; or at least one preservative or buffering agent selected from organic acid, citric acid, ascorbic acid, benzoic acid and their salts and derivatives, sodium benzoate, benzoate, hydroxybenzoate and derivatives, phosphate, sorbic acid, sodium sorbate, sorbate, salts, sodium chloride or potassium chloride, 1,2-Benzisothiazolin-3-one (BIT) or a combination thereof;
b. optionally at least one inhibitor selected from boric acid, boric acid derivative, aromatic borate ester, 4-formylphenyl boronic acid, phenyl boronic acid derivative, a peptide compound with inhibitorial function, or a combination thereof;
c. optionally at least one enzyme selected from protease, amylase, cellulase, lipase, xylanase, mannanase, cutinase, esterase, phytase, nuclease, pectinase, pectinolytic enzyme, pectate lyase, carbohydrase, arabinase, galactanase, xanthanase, xyloglucanase, laccase, peroxidase and oxidase with or without a mediator, or a combination thereof; and
d. optionally at least one filler selected from maltodextrin, flour, sodium chloride, sulfate, sodium sulfate, or a combination thereof.

According to an aspect is provided an enzyme composition comprising the variant of the first aspect and at least one stabilizer.

According to another aspect is provided an enzyme composition comprising the variant of the first aspect and at least one preservative, or comprising the variant of the first aspect and at least one buffering agent.

The present variant is suitable for use in various compositions. Because of the good stability of the variant, it can be used also in the presence of proteases even without protease inhibitors, such as inhibitors containing boron.

The components a-e provide improved properties for the present enzyme composition. The enzyme composition is compatible with the components a-e and improves applicability of the enzyme composition in various uses. Salts, such as sodium chloride and sodium sulfate function as drying aids.

According to the third aspect is provided a detergent composition comprising the present variant or the present enzyme composition.

An advantage of using the present variant in a detergent composition is that it performs well and remains stable for a long time. Advantageously it was discovered that compositions containing the present variant can be stored for a long time, and the mannan degrading activity is preserved in the presence of proteases even without boron or boron containing compounds. Thus, the present mannanase has good stability against proteases.

According to the fourth aspect is provided a recombinant host cell comprising genetic elements that allow producing at least one recombinant polypeptide comprising the variant of the first aspect.

According to the fifth aspect is provided a method for producing the variant of the first aspect, comprising cultivating the recombinant host cell of the fourth aspect, wherein:
the genetic elements comprise at least one control sequence which controls the production of the recombinant polypeptide in the recombinant host cell;
the genetic elements optionally comprise at least one sequence encoding a signal sequence for transporting the recombinant polypeptide outside the host cell; and
cultivating is carried out in conditions allowing production of the recombinant polypeptide.

According to the sixth aspect is provided a method for degrading or modifying mannan containing material comprising treating said mannan containing material with an effective amount of the enzyme composition of the second aspect or the variant of the first aspect.

In an embodiment the method comprises bringing the mannan containing material in contact with the enzyme composition or the variant in an aqueous medium.

According to the seventh aspect is provided an animal feed comprising the enzyme composition of the second aspect, or the variant of the first aspect, and at least one protein source of plant origin or a mannan containing product or by-product, and
a. Optionally at least one enzyme selected from protease, amylase, phytase, xylanase, endoglucanase, beta-glucanase, or a combination thereof; and
b. Optionally at least one filler selected from maltodextrin, flour, salt, sodium chloride, sulfate, sodium sulfate, or a combination thereof.

According to the eighth aspect is provided a feed supplement comprising the enzyme composition of the second aspect, or the variant of the first aspect; and
a. Optionally at least one enzyme selected from protease, amylase, phytase, xylanase, endoglucanase, beta-glucanase, or a combination thereof; and
b. Optionally at least one filler selected from maltodextrin, flour, salt, sodium chloride, sulfate, sodium sulfate or a combination thereof.

According to the ninth aspect is provided a use of the variant of the first aspect or the enzyme composition of the second aspect in oil drilling or hydro-fracturing; in processing of coffee extract, fruit juice, pineapple juice, or soya milk; in detergent; in degrading mannan containing stains; or in degrading mannan in an aqueous solution.

### Description of figures

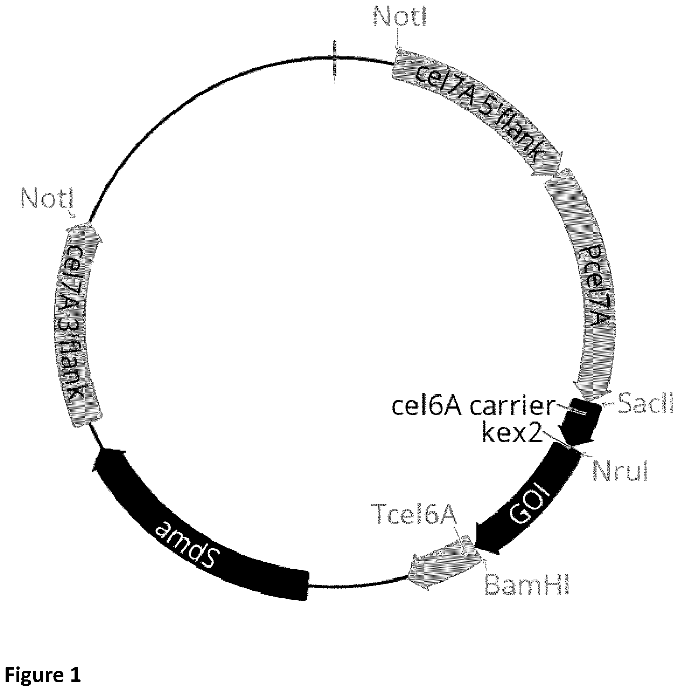

**Figure 1** shows a schematic picture of the expression plasmid used in the transformation of *Trichoderma reesei* for expression of the gene of interest (GOI) / mannanase variant genes. The expression of the recombinant genes in the host cell was controlled by the use of the following genetic elements: *T. reesei* cel7A promoter (Pcel7A) for transcription initiation, and *T. reesei* cel6A (Tcel6A) terminator for transcription termination. *T. reesei* cel6A carrier was used instead of the native mannanase signal sequence with kex2 as the cleavage site. The amdS gene (amdS) was included for selection of the transformants and *T. reesei* cel7A 3'- and 5'-flanking regions were used to optionally target the expression to cel7A locus. Picture was generated using Geneious Prime 2019 created by Biomatters.

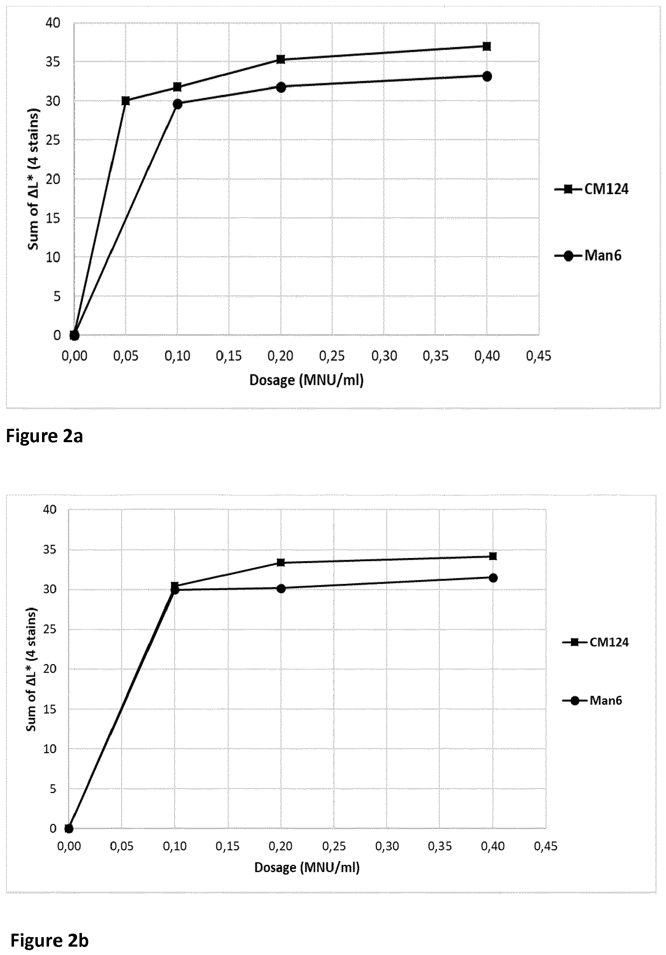

**Figure 2a** describes the stain removal performance of variant CM124 and parent Man6 mannanase as an increase of lightness (sum of ΔL* of 4 stains) in the presence of 4.4 g /l of commercial heavy duty liquid detergent at 40°C, 16°dH, 60 min, pH approx. 8.2 in Launder-Ometer. Enzymes were dosed as activity units (MNU) per ml of wash liquor.
**Figure 2b** describes the stain removal performance of variant CM124 and parent Man6 mannanase as an increase of lightness (sum of ΔL* of 4 stains) in the presence of 3.8 g /l of commercial bleach detergent powder at 40°C, 16°dH, 60 min, pH approx. 10 in Launder-Ometer. Enzymes were dosed as activity units (MNU) per ml of wash liquor.

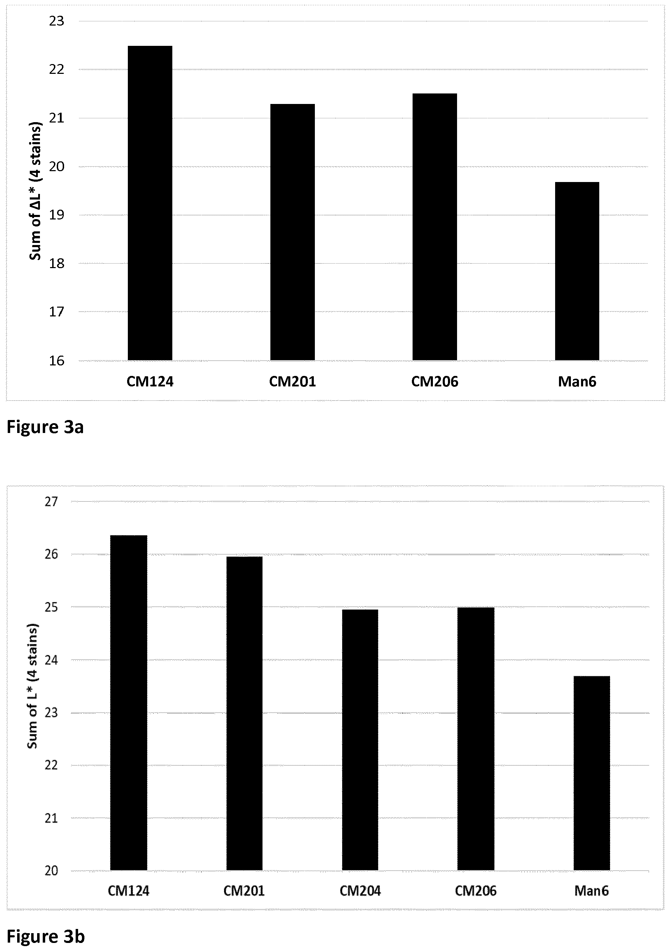

**Figure 3a** describes the stain removal performance of variants CM124, CM201, CM206 and parent Man6 mannanase as an increase of lightness (sum of ΔL* of 4 stains) in the presence of 3.2 g /l of commercial HDL Base detergent concentrate at 40°C, 16°dH, 60 min, pH approx. 8.4 in Launder-Ometer. Enzymes were dosed 0.8 activity units (MNU) per ml of wash liquor.
**Figure 3b** describes the stain removal performance of variants CM124, CM201, CM204, CM206 and parent Man6 mannanase as an increase of lightness (sum of ΔL* of 4 stains) in the presence of 3.8 g/l of commercial bleach detergent powder at 40°C, 16°dH, 60 min, pH approx. 10 in Launder-Ometer. Enzymes were dosed 0.8 activity units (MNU) per ml of wash liquor.

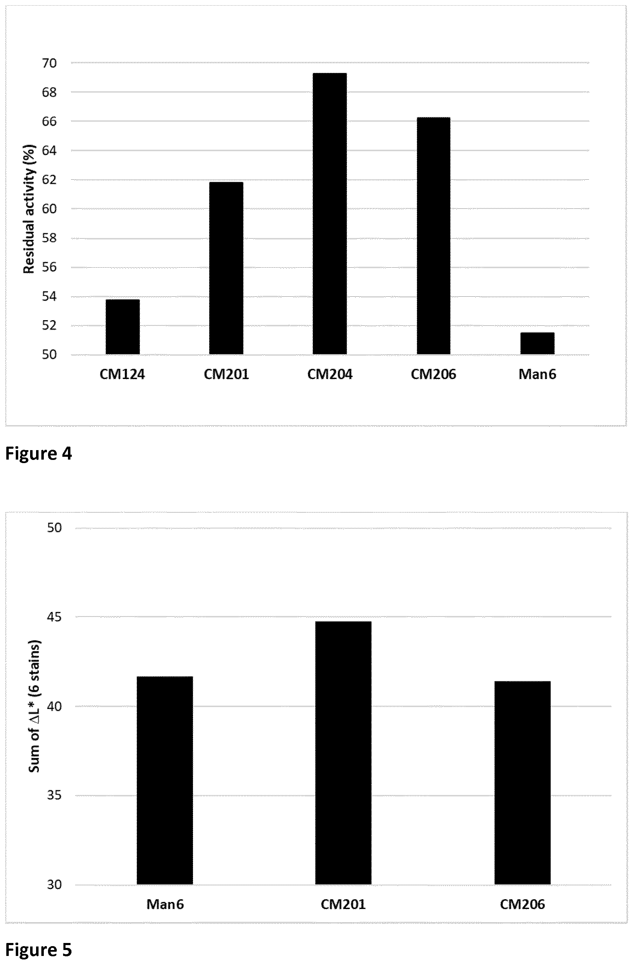

**Figure 4** shows the stability of variants CM124, CM201, CM204, CM206 and parent Man6 mannanase in protease containing commercial liquid detergent concentrate formulation without boric acid at 37°C and 4 weeks.
**Figure 5** describes the stain removal performance of variants CM201, CM206 and parent Man6 mannanase as an increase of lightness (sum of ΔL* of 6 stains) in full scale trials in washing machine at 40°C and 16°dH. Commercial liquid detergent concentrate with boric acid was dosed 56.9 g per wash. Enzymes were dosed 0.5 activity units (MNU) per ml of wash liquor.

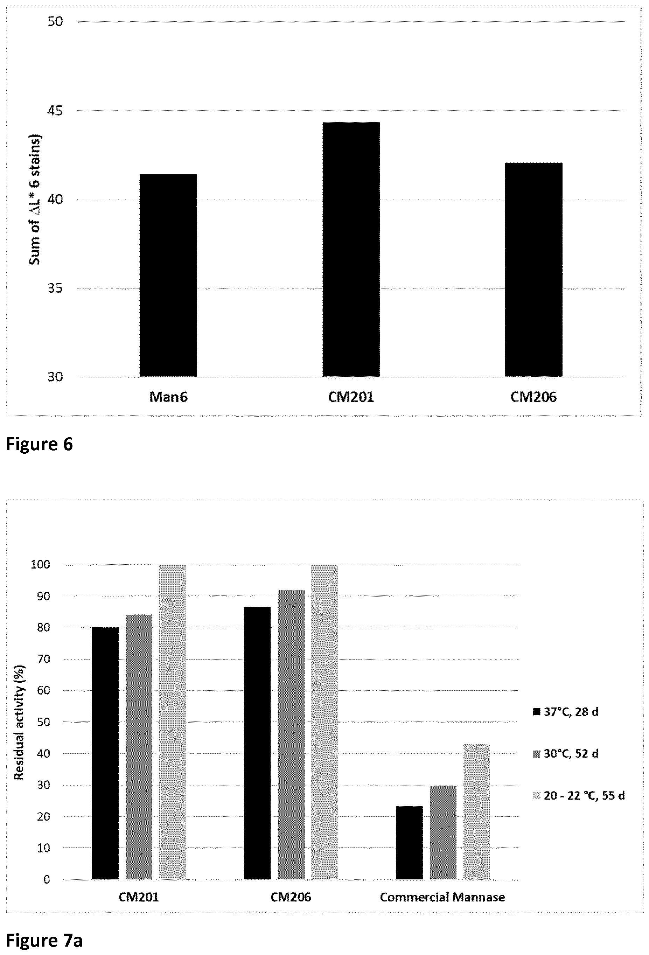

**Figure 6** describes the stain removal performance of variants CM201, CM206 and parent Man6 mannanase as an increase of lightness (sum of ΔL* of 6 stains) in full scale trials in washing machine at 40°C and 16°dH. Commercial bleach detergent powder was dosed 58.9 g per wash. Enzymes were dosed 0.5 activity units (MNU) per ml of wash liquor.
**Figure 7a** shows the stability of variants CM201 and CM206 compared to a commercial mannanase in protease containing commercial liquid detergent concentrate containing boric acid at 37°C and 4 weeks.

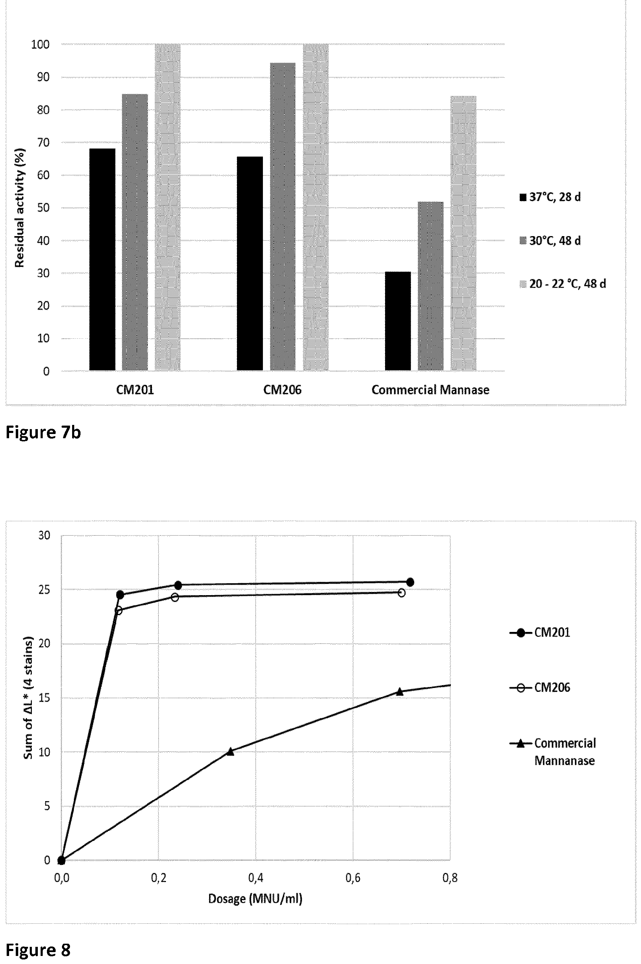

**Figure 7b** shows the stability of variants CM201 and CM206 compared to a commercial mannanase in protease containing commercial liquid detergent concentrate without boric acid at 37°C and 4 weeks.
**Figure 8** describes the stain removal performance of variants CM201 and CM206 as an increase of lightness (sum of ΔL* of 4 stains) in the presence of 3.8 g /l of commercial bleach detergent powder at 40°C, 16°dH, 60 min, pH approx. 10 in Launder-Ometer. Commercial mannanase was used for comparison. Enzymes were dosed as activity units (MNU) per ml of wash liquor.

### Sequence listings

SEQ ID NO: 1 amino acid sequence of the mature Man6 protein without signal peptide
SEQ ID NO: 2 nucleotide sequence of the *T. reesei* codon optimized *man6* gene without signal peptide encoding sequence

### Detailed description

In an embodiment at least position S256 of SEQ ID NO: 1 is substituted. Preferably the variants have improved performance over the parent mannanase Man6, to which substitutions are made. As can be seen from the Examples below, and from Figures 3a and 3b, the position 256 can be substituted with different amino acids and obtain an improved variant. E.g. the variants CM201 and CM204 having different substitutions in the position 256 both have improved performance over the parent Man6 enzyme. Different amino acid substitutions can thus be used in the position 256 as long as the variant retains mannanase activity, which is easily tested e.g. by using the test of Example 2. For example, variants CM125, CM79, CM80 and CM81 having different substitutions in position 256 have mannanase activity. Consequently, substitution of the residue 256 to Ala or Gly is a preferred embodiment of a substitution, but other substitutions can also be used.

The residual activity of the variants CM201 and CM204 was also improved over the parent Man6 enzyme in stability tests in detergents (see Example 4 and Figure 4). The results confirm that different substitutions in the position 256 can be used and, and storage stability of such variants can be improved further by modifying the position 24 by substitutions.

In an embodiment the variant comprises at least the following substitutions: positions 24 and 256; positions 24, 123 and 256; or positions 123 and 256. An advantage of these embodiments is improved performance over the parent mannanase. Another advantage is improved stability over a variant having a substitution in position 256, such as S256.

In an embodiment the variant has improved performance compared to a mannanase having the amino acid sequence of SEQ ID NO: 1. The performance can be measured as described in Example 2

In an embodiment the variant has improved stability compared to a mannanase having the amino acid sequence of SEQ ID NO: 1. The stability can be measured as described in Example 4.

In an embodiment the variant has improved stability in a protease containing detergent without a protease inhibitor, compared to a mannanase having the amino acid sequence of SEQ ID NO: 1.

In an embodiment the variant comprises at least the following substitutions: positions V24I and S256A; or positions V24I and S256G; or positions V24I, V123I, S256A; or positions V24I, V123I, and S256G; or positions V123I and S256A; or positions V123I and S256G.

In an embodiment the total number of substitutions in the variant compared to SEQ ID NO: 1 is 1-10, 2-10, 3-10, or 4-10. In an embodiment the variant has 1 substitution. In an embodiment the variant has 2 substitutions. In an embodiment the variant has 3 substitutions. In an embodiment the variant has 4 substitutions. In an embodiment the variant has 5 substitutions.

In an embodiment the variant is a variant disclosed in Table 1. These variants can be produced as recombinant proteins e.g. in a *Trichoderma* host cell.

In an embodiment the enzyme composition is provided in the form of a liquid composition or a solid composition, solution, dispersion, paste, powder, granule, granulate, coated granulate, tablet, cake, crystal, crystal slurry, gel, or pellet.

In an embodiment the detergent composition is provided in the form of a liquid detergent or a solid detergent, a bar, a homogenous tablet, a tablet having two or more layers, a pouch having one or more compartments, a regular or compact powder, a granule, a granulate, a paste, a gel, or a regular, compact or concentrated liquid.

In an embodiment the detergent composition further comprises one or more additional enzyme selected from protease, lipase, cutinase, amylase, carbohydrase, cellulase, pectinase, pectatelyase, pectinolytic enzyme, esterase, mannanase, arabinase, galactanase, xylanase, oxidase, xanthanase, xyloglucanase, laccase, nuclease and/or peroxidase, preferably selected from the group consisting of proteases, amylases, cellulases and lipases.

In an embodiment the detergent composition does not contain protease inhibitors, boric acid or its derivatives, such as borates and 4-formylphenyl boronic acid (4-FPBA), or peptide compounds with protease inhibitory function.

Mannan refers to polysaccharides consisting of a mannose backbone linked together by β-1,4-linkages with side-chains of galactose attached to the backbone by α-1,6-linkages. Mannans comprise plant-based material such as guar gum and locust bean gum. Glucomannans are polysaccharides having a backbone of more or less regularly alternating β-1,4 linked mannose and glucose, galactomannans and galactoglucomannans are mannans and glucomannans with alpha-1,6 linked galactose side branches.

As used herein, the term "mannanase" or "galactomannanase" means a mannanase enzyme defined in the art as mannan endo-1,4-beta-mannosidase and having the alternative names beta-mannanase and endo-1,4-mannanase and catalysing hydrolysis of 1,4-beta-D-mannosidic linkages in mannans, galactomannans, glucomannans, and galactoglucomannans. Mannanases are classified according to the Enzyme Nomenclature as EC 3.2.1.78.

As used herein, "isolated" means a substance in a form or environment that does not occur in nature. Non-limiting examples of isolated substances include (1) any non-naturally occurring substance, (2) any substance including any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing or decreasing the amount of the substance relative to other components with which it is naturally associated (e.g., recombinant production in a host cell; one or multiple copies of a gene encoding the substance; and use of an alternative promoter to the promoter naturally associated with the gene encoding the substance). In an embodiment the variant, polypeptide, enzyme, polynucleotide, host cell or composition of the invention is isolated.

As used herein, the term "comprising" includes the broader meanings of "including", "containing", and "comprehending", as well as the narrower expressions "consisting of" and "consisting only of".

The term variant means a polypeptide which has mannanase activity and which comprises an alteration at one more amino acid position(s). The alteration is preferably a substitution, insertion, and/or deletion, at one or more (e.g., several) positions. A substitution means replacement of the amino acid occupying a position with a different amino acid, or with different amino acids; a deletion means removal of the amino acid occupying a position; and an insertion means adding an amino acid adjacent to and immediately following the amino acid occupying a position. In an embodiment the variants of the present invention have at least 80%, e.g., at least 80, 81, 82, 83, 84, 85, 86, 87, 88, 89, 90, 91, 92, 93, 94, 95, 96, 97, 98, or 99% sequence identity with SEQ ID NO: 1. In an embodiment the variant does not have 100% sequence identity with the SEQ ID NO: 1.

In an embodiment the improved performance of the variant comprises increased mannan degrading activity compared to the parent mannanase to which at least one substitution is made. Mannan degrading activity can be determined by using the assay described in the Examples below.

Preferably, the stain removal or wash performance of the variant is similar or improved compared to the parent or reference mannanase. The wash performance can be measured according to the Examples below.

Parent or parent mannanase means a mannanase to which an alteration is made to produce the variant of the present invention. The parent may be a naturally occurring (wild-type) polypeptide or a variant thereof, or a fragment thereof. In a preferable embodiment the parent comprises a polypeptide having the amino acid sequence of SEQ ID NO: 1. In a preferred embodiment the parent mannanase is a bacterial mannanase having at least 90%, preferably 91, 92, 93, 94, 95, 96, 97, 98 or 99%, sequence identity with SEQ ID NO: 1.

As used herein, a "peptide" and a "polypeptide" are amino acid sequences including a plurality of consecutive polymerized amino acid residues. For purpose of this invention, peptides are molecules including up to 20 amino acid residues, and polypeptides include more than 20 amino acid residues. The peptide or polypeptide may include modified amino acid residues, naturally occurring amino acid residues not encoded by a codon, and non-naturally occurring amino acid residues. As used herein, a "protein" may refer to a peptide or a polypeptide of any size. A protein may be an enzyme, a protein, an antibody, a membrane protein, a peptide hormone, regulator, or any other protein.

For an amino acid substitution, the following nomenclature is used: original amino acid, position, substituted amino acid. Accordingly, the substitution of serine at position 256 with alanine is designated as "Ser256Ala" or "S256A".

Preferably the amino acid numbering of the variant corresponds to the numbering of SEQ ID NO: 1. The amino acid numbering of two polypeptides can be considered to be corresponding when their sequences are aligned.

The term "polynucleotide" means a single- or double-stranded polymer of deoxyribonucleotide or ribonucleotide bases read from the 5' to the 3' end. Polynucleotides include RNA and DNA, and may be isolated from natural sources, synthesized in vitro, or prepared from a combination of natural and synthetic molecules.

As used herein, "identity" means the percentage of exact matches of amino acid residues between two aligned sequences over the number of positions where there are residues present in both sequences. When one sequence has a residue with no corresponding residue in the other sequence, the alignment program allows a gap in the alignment, and that position is not counted in the denominator of the identity calculation. Identity is a value determined with the Pairwise Sequence Alignment tool EMBOSS Needle at the EMBL-EBI website (www.ebi.ac.uk/Tools/psa/emboss_needle/).

As used herein, "host cell" means any cell type that is susceptible to transformation, transfection, transduction, mating, crossing or the like with a nucleic acid construct or expression vector comprising a polynucleotide. The term "host cell" encompasses any progeny that is not identical due to mutations that occur during replication. Non-limiting examples of useful host cells are fungal cells, filamentous fungal cells from Division *Ascomycota,* Subdivision *Pezizomycotina*; preferably from the group consisting of members of the Class *Sordariomycetes,* Subclass *Hypocreomycetidae,* Orders *Hypocreales* and *Microascales* and *Aspergillus, Chrysosporium, Myceliophthora* and *Humicola*; more preferably from the group consisting of Families *Hypocreacea, Nectriaceae, Clavicipitaceae, Microascaceae,* and Genera *Trichoderma* (anamorph of *Hypocrea), Fusarium, Gibberella, Nectria, Stachybotrys, Claviceps, Metarhizium, Villosiclava, Ophiocordyceps, Cephalosporium,* and *Scedosporium*; more preferably from the group consisting of *Trichoderma reesei (Hypocrea jecorina), T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Gibberella fujikuroi, G. moniliformis, G. zeaea, Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum,* and *Scedosporium apiospermum,* and *Aspergillus niger, Aspergillus awamori, Aspergillus oryzae, Chrysosporium lucknowense, Myceliophthora thermophila, Humicola insolens,* and *Humicola grisea,* most preferably *Trichoderma reesei.*

Non-limiting examples of a host cell are bacterial cells, preferably gram-positive *Bacilli* (e.g. *Bacillus subtilis, B. licheniformis, B. megaterium, B. amyloliquefaciens, B. pumilus),* gram-negative bacteria (e.g. *Escherichia coli*), actinomycetales (e.g. *Streptomyces sp*.) and yeasts (e.g. *Saccharomyces cerevisiae, Pichia pastoris, Yarrowia lipolytica).*

In an embodiment the host cell is a fungal cell, preferably a filamentous fungal cell, such as *Trichoderma* or *Trichoderma reesei.* In an embodiment the host cell is a bacterial cell, preferably a gram-positive *Bacillus* cell, such as *B*. *subtilis, B. licheniformis, B. megaterium, B. amyloliquefaciens, B. pumilus.*

A "recombinant cell" or "recombinant host cell" refers to a cell or host cell, which has been genetically modified or altered to comprise a nucleic acid sequence which is not native to said cell or host cell. In an embodiment the genetic modification comprises integrating the polynucleotide in the genome of the host cell. In another embodiment the polynucleotide is exogenous in the host cell.

As used herein, "expression" includes any step involved in the production of a polypeptide in a host cell including, but not limited to, transcription, translation, post-translational modification, and secretion. Expression may be followed by harvesting, i.e. recovering, the host cells or the expressed product.

The term "expression vector" means a DNA molecule, linear or circular, that comprises a segment encoding a polypeptide of interest operably linked to additional segments (genetic elements) that provide for its transcription. Such additional segments may include promoter and terminator sequences, and may optionally include one or more origins of replication, one or more selectable marker, an enhancer, a polyadenylation signal, carrier and the like. Expression vectors are generally derived from plasmid or viral DNA, or they may contain elements of both. The expression vector may be any expression vector that is conveniently subjected to recombinant DNA procedures, and the choice of vector will often depend on the host cell into which the vector is to be introduced. Thus, the vector may be an autonomously replicating vector, i.e. a vector, which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

The term "obtained from" and "obtainable" as used herein in connection with a specific microbial source means that the polynucleotide is expressed by the specific source (homologous expression), or by a cell in which a gene from the source has been inserted (heterologous expression).

The term "enzyme composition" means either a conventional enzymatic fermentation product, possibly isolated and purified, from a single species of a microorganism, such preparation usually comprising a number of different enzymatic activities; or a mixture of monocomponent enzymes, preferably enzymes derived from bacterial or fungal species by using conventional recombinant techniques, which enzymes have been fermented and possibly isolated and purified separately and which may originate from different species, preferably fungal or bacterial species or the fermentation product of a microorganism which acts as a host cell for production of a recombinant mannanase, but which microorganism simultaneously produces other enzymes.

The term "operably linked", when referring to DNA segments, means that the segments are arranged so that they function in concert for their intended purposes, e.g. transcription initiates in the promoter and proceeds through the coding segment to the terminator. The term "promoter" means a portion of a gene containing DNA sequences that provide for the binding of RNA polymerase and initiation of transcription. Promoter sequences are preferably in the 5' non-coding regions of genes.

The term "secretory signal sequence" or "signal sequence" means a DNA sequence that encodes a polypeptide (a "secretory peptide" or "signal peptide") that, as a component of a larger polypeptide, directs the larger polypeptide through a secretory pathway of a host cell in which it is produced. The secretory signal sequence can be native or it can be replaced with secretory signal sequence or carrier sequence from another source. Depending on the host cell, the larger peptide may be cleaved to remove the secretory peptide during transit through the secretory pathway.

The term "core region" means a domain of an enzyme, which may or may not have been modified or altered, but which has retained at least part of its original activity; the catalytic domain as known in the art has remained functional. The core region of a variant according to the invention corresponds to the amino acids aligned with the amino acids 1-289 of Man6, SEQ ID NO: 1.

Efficient amount means an amount of the variant which is sufficient to degrade mannan in the selected application or use.

Preferably the improved performance of a variant comprises the meaning of increased performance of the variant in degrading mannan in certain experimental conditions. Thus, the performance of the variant can be tested by the skilled person and compared with a reference, such as a wild type enzyme or another variant.

The terms "detergent composition" and "detergent" include, unless otherwise indicated, solid, granular or powder-form all-purpose or heavy-duty washing agents, especially cleaning detergents; liquid, gel or paste-form all-purpose washing agents, especially the so-called heavy-duty liquid (HDL) types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, car or carpet shampoos, bathroom cleaners; metal cleaners; as well as cleaning auxiliaries such as bleach additives and "stain-stick" or pre-treat types. The terms "detergent", "detergent composition" and "detergent formulation" are used in reference to mixtures, which are intended for use in a wash medium for the cleaning of soiled objects. In some embodiments, the term is used in reference to laundering fabrics and/or garments (e.g., "laundry detergents"). In alternative embodiments, the term refers to other detergents, such as those used to clean dishes, cutlery, etc. (e.g., "dishwashing detergents"). It is not intended that the present invention be limited to any particular detergent formulation or composition. It is intended that in addition to containing the mannanase variants according to the invention, the detergents may contain e.g. surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anticorrosion agents, hydrotropes, fabric hueing agents, dispersants, dye transfer inhibiting agents, fluorescent whitening agents, soil release polymers, anti-redepositions agents, anti-shrink agents, anti-wrinkling agents, binders, carriers, dyes, enzyme stabilizers, fabric softeners, fillers, foam regulators, perfumes, pigments, sod suppressors, solvents, and structurants for liquid detergents, structure elasticizing agents, enzyme inhibitors or stabilizers, enzyme activators, transferase(s), hydrolytic enzymes, oxido reductases, bluing agents and fluorescent dyes, antioxidants, and solubilizers. However, as discussed above, protease inhibitors are optional because the present variants are stable in the presence of proteases in several detergent formulations. Protease inhibitors may be useful in multi-enzyme compositions and detergents to protect other enzymes from degradation.

The term "textile" means any textile material including yarns, yarn intermediates, fibers, non-woven materials, natural materials, synthetic materials, and any other textile material, fabrics made of these materials and products made from fabrics (e.g., garments, linen and other articles). The textile or fabric may be in the form of knits, wovens, denims, non-wovens, felts, yarns, and towelling. The textile may be cellulose based, such as natural cellulosics including cotton, flax/linen, jute, ramie, sisal or coir or manmade cellulosics (e.g. originating from wood pulp) including viscose/rayon, ramie, cellulose acetate fibers (tricell), lyocell or blends thereof. The textile or fabric may also be non-cellulose based such as natural polyamides including wool, camel, cashmere, mohair, rabit and silk or synthetic polymer such as nylon, aramid, polyester, acrylic, polypropylen and spandex/elastane, or blends thereof as well as blend of cellulose based and non- cellulose based fibers. Examples of blends are blends of cotton and/or rayon/viscose with one or more companion material such as wool, synthetic fibers (e.g. polyamide fibers, acrylic fibers, polyester fibers, polyvinyl alcohol fibers, polyvinyl chloride fibers, polyurethane fibers, polyurea fibers, aramid fibers), and cellulose-containing fibers (e.g. rayon/viscose, ramie, flax/linen, jute, cellulose acetate fibers, lyocell). Fabric may be conventional washable laundry, for example stained household laundry. When the term fabric or garment is used it is intended to include the broader term textiles as well.

The term "stability" includes in certain embodiments storage stability and stability during use, e.g. during a wash process (in wash stability) and reflects the stability of the variant according to the invention as a function of time, e.g. how much activity is retained when the mannanase is kept in solution, in particular in a detergent solution. The stability is influenced by many factors, e.g. pH, temperature, detergent composition e.g. proteases, stabilizers, builders, surfactants, protease inhibitors etc. The mannanase stability may be measured using the 'activity assay' as described in examples.

"Mannanase activity" as used herein refers to the mannan degrading activity of a polypeptide, such as the present variant. Degrading or modifying as used herein means that mannose units are hydrolyzed from the mannan polysaccharide by the mannanase. The mannan degrading activity of the polypeptides according to present invention can be tested according to standard test procedures known in the art. Example 2 provides an example of a method for determining mannanase activity.

In one embodiment of the present invention the enzyme composition further comprises one or more additional enzymes selected from the group consisting of protease, lipase, cutinase, amylase, carbohydrase, cellulase, pectinase, pectatelyase, pectinolytic enzyme, esterase, phytase, mannanase, arabinase, galactanase, xylanase, oxidase, xanthanase, xyloglucanase, nuclease, laccase, and/or peroxidase, preferably selected from the group consisting of proteases, amylases, cellulases and lipases.

The present enzyme composition comprising the variant and an additional enzyme is advantageous in providing synergistic effect. Such additional enzymes are desired when the present enzyme composition comprising mannanase is used in detergents e.g. when washing stains. Particularly advantageous synergistic enzymes that work with mannanases and mannanase variants are amylases, proteases and cellulases, or a combination thereof, such as a composition comprising mannanase, amylase and protease.

In general the properties of the selected enzyme(s) should be compatible with the selected detergent, (i.e., pH-optimum, compatibility with other enzymatic and non-enzymatic ingredients, etc.), and the enzyme(s) should be present in effective amounts.

A composition for use in solid laundry detergent, for example, may include 0.000001% - 5%, such as 0.000005-2%, such as 0.00001%-1%, such as 0.00001%-0.1% of enzyme protein by weight of the composition. In an embodiment the enzyme protein is the variant of the present invention.

A composition for use in laundry liquid, for example, may include 0.000001%-3%, such as 0.000005%-1%, such as 0.00001%-0.1% of enzyme protein by weight of the composition. In an embodiment the enzyme protein is the variant of the present invention.

A composition for use in automatic dishwash, for example, may include 0.000001%-5%, such as 0.000005%-2%, such as 0.00001%-1%, such as 0.00001%-0.1% of enzyme protein by weight of the composition. In an embodiment the enzyme protein is the variant of the present invention.

In a further embodiment of the present invention the detergent composition is in the form of a bar, a homogenous tablet, a tablet having two or more layers, a pouch having one or more compartments, a regular or compact powder, a granule, a paste, a gel, or a regular, compact or concentrated liquid. In one embodiment the detergent composition can be a laundry detergent composition, preferably a liquid or solid laundry detergent composition. There are a number of detergent formulation forms such as layers (same or different phases), pouches, as well as forms for machine dosing unit.

### Examples

The following examples are provided to illustrate various aspects of the present invention. They are not intended to limit the invention, which is defined by the accompanying claims.

### Example 1. Variant design and expression of synthetic mannanase variants

Standard molecular biology methods were used in the isolation and enzyme treatments of DNA (e.g. isolation of plasmid DNA, digestion of DNA to produce DNA fragments), in *E. coli* transformations, sequencing etc. The basic laboratory methods used were either as described by the enzyme, reagent or kit manufacturer or as described in the standard molecular biology handbooks, e.g. Sambrook and Russell (2001) or as described in the following examples.

To improve the performance of the *Bacillus clausii* Man6 mannanase, 210 variants were designed based on structural analysis of wild type enzyme (Table 1). Man6 mannanase variants were derived from a parent Man6 molecule, which is the wild type *Bacillus clausii* Man6 mannanase without its native signal peptide (SEQ ID NO: 1) produced in *Trichoderma reesei.* The nucleic acid sequence encoding the parent mannanase was codon optimised for *T. reesei* (SEQ ID NO: 2). Expression vectors (plasmids) were constructed for production of recombinant mannanase variants CM1-210 using a carrier sequence from *T. reesei* cel6A instead of the native signal sequence of *Bacillus clausii* Man6 mannanase. The expression constructs contain operably linked *T. reesei* cel7A promoter, cel6A CBD as carrier, kex2 cleavage site, mannanase variant gene and cel6A terminator, followed by the amdS marker gene as described in Paloheimo et al. 2003. In addition, the constructs contain cel7A 3' and 5' flanking regions for optionally targeting the expression vector into the cel7A locus (Figure 1). Synthetic genes, including mutations introduced in the core region of the parent molecule, were exactly fused as Nrul-BamHI fragments to the cel6A carrier/kex2 reading frame after the *T. reesei* cel7A promoter by ligation.

Circular expression plasmids were used in *T. reesei* protoplast transformation. The transformants were selected with acetamide as the sole nitrogen source. The host strain lacks the four major endogenous cellulases: CBHI/Cel7A, CBHII/Cel6A, EGI/Cel7B and EGII/Cel5A. The transformations were performed according to Penttilä et al, 1987, with the modifications described in Karhunen et al., 1993. The transformants were sporulated on potato dextrose agar (PDA) prior to shake flask cultivation.

The mannanase production of the transformants was analyzed from the culture supernatants of shake flask cultivations (50 ml). The transformants were grown for 7 days at 30°C, 250 rpm in a complex cellulase-inducing medium (Joutsjoki et al., 1993) buffered with 5% KH₂PO₄ at pH 6.0. The enzyme activity of the recombinant protein was measured from the culture supernatant as the release of reducing sugars from galactomannan as described in Example 2. Production of the recombinant protein was also detected from the culture supernatant by sodium dodecyl sulfate polyacrylamide gel electrophoresis (SDS-PAGE).

Chosen transformants and the reference strain producing the parent Man6 mannanase were purified on selection plates through single conidia prior to sporulating them on PDA and cultivated in shake flasks or bioreactors in complex cellulase-inducing medium to obtain material for the application tests (Examples 3 to 7).

**Table 1. List of Man6 variants produced in Trichoderma reesei. The amino acid numbering corresponds to the amino acid numbering of the parent molecule presented in SEQ ID NO: 1.**

| | |
|---|---|
| CM1 | S7N |
| CM2 | E10N |
| CM3 | E10T |
| CM4 | E10K |
| CM5 | L12N |
| CM6 | D17A |
| CM7 | D17T |
| CM8 | D17S |
| CM9 | D17K |
| CM10 | D17N |
| CM11 | D17E |
| CM12 | D17Q |
| CM13 | Y19F |
| CM14 | V24I |
| CM15 | F31Y |
| CM16 | Q33H |
| CM17 | E36N |
| CM18 | E36Q |
| CM19 | E36T |
| CM20 | E36A |
| CM21 | E36Y |
| CM22 | E44N |
| CM23 | E44Q |
| CM24 | E44K |
| CM25 | E44A |
| CM26 | V50I |
| CM27 | T75V |
| CM28 | T82A |
| CM29 | T82P |
| CM30 | T82V |
| CM31 | D93N |
| CM32 | D94S |
| CM33 | D94N |
| CM34 | D94E |
| CM35 | P95I |
| CM36 | P95A |
| CM37 | P95V |
| CM38 | P95T |
| CM39 | P95Y |
| CM40 | P95L |
| CM41 | P95M |
| CM42 | P95R |
| CM43 | K100R |
| CM44 | A109K |
| CM45 | A109R |
| CM46 | T115R |
| CM47 | V123I |
| CM48 | S133G |
| CM49 | S133A |
| CM50 | S133T |
| CM51 | L152I |
| CM52 | S153R |
| CM53 | V158I |
| CM54 | A168Q |
| CM55 | A168S |
| CM56 | E172D |
| CM57 | E172Q |
| CM58 | R173Y |
| CM59 | R173F |
| CM60 | R173E |
| CM61 | A175Q |
| CM62 | A175R |
| CM63 | A175K |
| CM64 | D176S |
| CM65 | D176A |
| CM66 | K184R |
| CM67 | T186I |
| CM68 | D199N |
| CM69 | T202M |
| CM70 | T202Q |
| CM71 | A204Q |
| CM72 | A204R |
| CM73 | E213Q |
| CM74 | V218I |
| CM75 | A238S |
| CM76 | A238R |
| CM77 | A241Q |
| CM79 | S256N |
| CM80 | S256R |
| CM81 | S256H |
| CM82 | V259L |
| CM83 | A265S |
| CM84 | E266Y |
| CM85 | E266V |
| CM86 | S269Q |
| CM87 | K278Q |
| CM88 | K278T |
| CM89 | K278R |
| CM90 | E284P |
| CM91 | K288R |
| CM92 | S289Q |
| CM93 | D290T |
| CM94 | T75V, A175R, E213Q |
| CM95 | T82V, V123L, T186I |
| CM96 | V123L, V158I, V218I |
| CM97 | F31Y, D93N, S256G |
| CM98 | F31Y, S256G, H227T |
| CM99 | F31Y, S130A, S256G |
| CM100 | E10T, P95R, A109R, A141I |
| CM101 | E10T, A141I, K184R, A204R |
| CM 102 | E213Q, A238R, E266Y, K288R |
| CM103 | A109R, K184R, A238R |
| CM104 | R225M, H227T, S256G, G257A, G258Q, V259W |
| CM105 | V24L |
| CM 106 | F31V |
| CM107 | V123L |
| CM 108 | V217I |
| CM 109 | R225A |
| CM110 | R225N |
| CM111 | R225D |
| CM112 | R225E |
| CM113 | R225L |
| CM114 | R225S |
| CM115 | H226W |
| CM116 | H226F |
| CM117 | H226A |
| CM118 | H226S |
| CM119 | H226P |
| CM120 | H226E |
| CM121 | D228A |
| CM122 | D228S |
| CM123 | S256A |
| CM124 | S256G |
| CM125 | S256T |
| CM126 | V259I |
| CM127 | V259F |
| CM128 | V259Y |
| CM129 | V259A |
| CM130 | V259N |
| CM131 | V259D |
| CM132 | V259E |
| CM133 | V259S |
| CM134 | V259T |
| CM135 | V259G |
| CM136 | Y261A |
| CM137 | Y261Q |
| CM138 | Y261S |
| CM139 | L262N |
| CM140 | L262A |
| CM141 | L262S |
| CM142 | L262Q |
| CM143 | D228A, V259G, L262N |
| CM144 | H226S, V259D, L262Q |
| CM145 | V24I, V123I, V259F |
| CM146 | V123I, H226F, S256G |
| CM147 | F31V, H226A, S256G |
| CM148 | F31V, V259G, L262S |
| CM149 | R225S, V259E, L262S |
| CM150 | V123L, H226E, S256A |
| CM151 | R225L, H226P, V259I |
| CM152 | R225A, S256A, Y261Q |
| CM153 | H226S, S256G, V259L |
| CM154 | H226S, V259N, L262A |
| CM155 | H226P, V259F, L262N |
| CM156 | S256T, V259S, Y261A |
| CM157 | V217I, V259A, L262Q |
| CM158 | R225D, D228S, V259Y |
| CM159 | R225E, V259T, Y261Q |
| CM160 | V217I, V259D, L262N |
| CM161 | R225D, H226F, V259F |
| CM162 | S256A, V259N, L262S |
| CM163 | R225E, D228A, V259A |
| CM164 | R225L, V259S, L262A |
| CM165 | F31V, R225S, V259Y |
| CM166 | V123I, R225D, V259A |
| CM167 | V123L, R225L, V259E |
| CM168 | R225N, H226A, V259G |
| CM169 | V24L, H226W, L262S |
| CM170 | V24I, V259I, L262A |
| CM171 | V24I, H226S, S256A |
| CM172 | R225S, H226P, V259D |
| CM173 | V123L, H226F, L262A |
| CM174 | R225A, H226W, V259F |
| CM175 | R225A, D228S, V259S |
| CM176 | V24L, R225S, V259I |
| CM177 | H226P, V259T, Y261A |
| CM178 | H226F, Y261S, L262Q |
| CM179 | R225N, H226W, V259L |
| CM180 | R225N, H226E, Y261Q |
| CM181 | R225L, D228S, L262N |
| CM182 | V24L, D228A, V259D |
| CM183 | D228S, S256T, V259E |
| CM184 | S256G, V259G, Y261A |
| CM185 | V217I, R225E, Y261S |
| CM186 | V24L, R225D, V259L |
| CM187 | V259E, Y261Q |
| CM188 | F31V, R225N, V259A |
| CM189 | V123L, V259T |
| CM190 | H226W, V259I |
| CM191 | D228A, V259T, Y261S |
| CM192 | V217I, H226E, V259Y |
| CM193 | R225A, H226E, V259L |
| CM194 | S256T, V259N |
| CM195 | Y261A, L262Q |
| CM196 | H226A, V259S |
| CM197 | V24I, V259N |
| CM198 | V123I, S256T |
| CM199 | R225E, H226A |
| CM200 | V259Y, Y261S |
| CM201 | S256G, V24I |
| CM202 | S256G, V123I |
| CM203 | S256G, V24I, V123I |
| CM204 | S256A, V24I |
| CM205 | S256A, V123I |
| CM206 | S256A, V24I, V123I |
| CM207 | D228S, S256G |
| CM208 | D228S, S256G, V24I |
| CM209 | D228S, S256G, V123I |
| CM210 | D228S, S256G, V24I, V123I |

### Example 2. Assay of galactomannanase activity by DNS -method

Mannanase activity (MNU) was measured as the release of reducing sugars from galactomannan (0.3 w/w-%) at 50°C and pH 7.0 in 5 min. The amount of released reducing sugars was determined spectrophotometrically using dinitrosalicylic acid.

Substrate (0.3 w/w-%) used in the assay was prepared as follows: 0.6 g of locust bean gum (Sigma G-0753) was dissolved in 50 mM sodium citrate buffer pH 7 (or citrate phosphate buffer pH 7) at about 80°C using a heating magnetic stirrer and heated up to boiling point. The solution was cooled and let to dissolve overnight in a cold room (2 - 8°C) with continuous stirring and insoluble residues were removed by centrifugation. After that, solution was filled up to 200 ml with buffer. Substrate was stored frozen and melted by heating in a boiling water bath to about 80°C, cooled to room temperature and mixed carefully before use.

DNS reagent used in the assay was prepared by dissolving 50 g of 3.5- dinitrosalisylic acid (Sigma D-550) in about 4 liters of water. With continuous magnetic stirring 80.0 g of NaOH was gradually added and let to dissolve. An amount of 1500 g of Rochelle Salt (K-Na-tartrate, Merck 8087) was added in small portions with continuous stirring. The solution was cautiously warmed to a maximum temperature of 45°C, cooled to room temperature and filled up to 5000 ml. After that, it was filtered through Whatman 1 filter paper and stored in a dark bottle at room temperature.

The reaction was first started by adding 1.8 ml of substrate solution to each of the two test tubes and let to equilibrate at 50°C for 5 minutes, after which 200 µl of suitably diluted enzyme solution was added to one of the tubes, mixed well with vortex mixer and incubated exactly for 5 min at 50°C. Enzyme blanks were not equilibrated or incubated. The reaction was stopped by adding 3.0 ml of DNS reagent into both tubes and mixed. After DNS addition, 200 µl of sample solution was added to the enzyme blank tubes. Both tubes were placed in a boiling water bath. After boiling for exactly 5 minutes, the tubes were placed in a cooling water bath and allow them to cool to room temperature. The absorbance of sample was measured against the enzyme blank at 540 nm and activity was read from the calibration curve and multiplied by the dilution factor. A suitable diluted sample yielded a target absorbance difference of 0.3 - 0.45.

Standard curve was prepared from 20 mM mannose stock solution by dissolving 360 mg of mannose (Sigma M-6020, stored in a desiccator) in 100 ml assay buffer and diluted to solutions containing 3, 6, 10 and 14 µmol/ml of mannose. Standards were handled like the samples except for incubating at 50°C. The absorbances were measured against the reagent blank (containing buffer instead of standard dilution of mannose) at 540 nm. Calibration curve was constructed for every series of assays.

One mannanase unit (MNU) was defined as the amount of enzyme that produces reductive sugars having a reductive power corresponding to one nmol of mannose from galactomannan in one second under the assay conditions (1 MNU = 1nkat).

### Example 3. Stain removal performance of mannanase variants CM124, CM201, CM204 and CM206 compared to parent Man6 mannanase in small scale tests at 40°C in Launder-Ometer

Shake flask supernatants of mannanase variants CM124, CM201, CM204, and CM206 produced in *Trichoderma* (as described in Example 1) were tested for their ability to remove mannanase sensitive standard stains at 40 °C and water hardness of 16°dH with commercial detergents. Parent Man6 was used as reference. The following artificially soiled test cloths from Center for testmaterial B.V. (the Netherlands) were used: Chocolate pudding mannanase sensitive on cotton (E-165), Locust bean gum, with pigment on cotton (C-S-73), Guar gum with carbon black on cotton (C-S-43) and Mayonnaise, with carbon black on cotton (C-S-05S). The fabric was cut in 6 cm x 6 cm swatches and 2 pieces of each were used in test.

Commercial heavy duty liquid detergent (HDL) described in Table 2, was used at concentration of 4.4 g per liter of wash liquor, commercial HDL base detergent concentrate was used at 3.2 g/l and commercial bleach detergent powder was used at 3.8 g/l. Detergents were containing all other enzymes except mannanase. Detergent containing wash liquors was prepared in synthetic tap water with hardness of 16°dH. pH of the wash liquor of liquid detergents was approximately 8.2 - 8.4, and with the bleach detergent approx. 10.

**Table 2. Composition of commercial liquid detergent**

| **Ingredient** | % | |
|---|---|---|
| Anionic surfactants, soap | 15 - 30 | |
| Nonionic surfactants | 5 - 15 | |
| Phosphonate, | < 5 | |
| Boric acid | ≤1 | |
| Other ingredients: e.g. optical brighteners, perfumes, preservatives | | |
| pH 8.2-8.6 | | |

Mannanases were dosed as 0.05 - 0.8 MNU activity per ml of wash liquor depending on tests and detergent. Activity was measured as described in Example 2. Control sample contained the detergent solution without mannanase.

For synthetic tap water with hardness of 16°dH the following stock solutions were prepared in deionized water (Milli-Q or equivalent):
Stock solution with 1000°d Calcium-hardness: CaCl₂ x 2 H₂O (1.02382.1000, Merck KGaA, Germany) 26.22 g/l
Stock solution with 200°d Magnesium-hardness: MgSO₄ x 7 H₂O (1.05886.1000, Merck KGaA, Germany) 8.79 g/l
NaHCO₃ stock solution: NaHCO₃ (1.06329.1000 Merck KGaA, Germany) 29.6 g/l

13.3 ml CaCl₂ solution, 13.3 ml MgSO₄ solution and 10.0 ml of freshly made Na-HCO₃ solution were added in volumetric flask in the given order, made up to 1 liter with deionized water and mixed. The hardness of water was determined by complexometric titration and found correct.

Stain removal treatments were performed in Atlas LP-2 Launder-Ometer as follows. Launder-Ometer was first preheated to 40°C. Then detergent, 250 ml synthetic tap water with hardness of 16°dH and diluted enzyme (<1.0 ml) were added into 1.2-liter containers. Stains were added and the Launder-Ometer was run at 40°C for 60 min with a rotation speed of 42 rpm. After that the swatches were carefully rinsed under running water and dried overnight at indoor air, on a grid protected against daylight.

The stain removal effect was evaluated by measuring the colour as reflectance values with Konica Minolta CM-3610A spectrophotometer using L*a*b* color space coordinates (illuminant D65/10°, 420 nm cut). Fading of the stains, indicating mannanase performance (stain removal effect/efficiency) was calculated as ΔL* (delta L*), which means lightness value L* of enzyme treated fabric minus lightness value L* of fabric treated with washing liquor without mannanase (control). Final results (total stain removal effect) were shown as sum of ΔL* of each 4 stains. Color values of each stains were average of 2 swatches.

Variant CM124 was compared to parent Man6 in commercial heavy duty liquid detergent (Figure 2a) and commercial bleach detergent powder (Figure 2b) using dosages 0 - 0.4 MNU per ml of wash liquor. CM124 had improved stain removal performance compared to parent Man6 in both detergents.

Further tests were carried out with variants CM124, CM201 and CM206 in commercial HDL base detergent concentrate (Figure 3a) and commercial bleach detergent powder (Figure 3b) using parent Man6 as reference. Mannanase was dosed 0.8 MNU/ml in both tests, and in bleach detergent also variant CM204 was tested. Based on results all the variant mannanases had better stain removal performance than parent Man6.

### Example 4. Stability of mannanase variants (CM124, CM201, CM204, CM206) compared to parent Man6 in liquid detergent

The stability of the best performing mannanase variants was compared to parent Man6 mannanase. Shake flask cultivation supernatants of variants CM124, CM201, CM204 and CM206, produced in *Trichoderma* (as described in Example 1), were added 4 w/w-% in detergent. Commercial liquid detergent concentrate without boric acid was used. It contained other enzymes including protease, but no mannanase Samples were incubated in plastic tubes with caps at 37°C for 4 weeks. The activity was measured at certain intervals by activity assay described in Example 2 except using 30 min incubation time. Results were calculated as residual activity (%), which was obtained by dividing the activity of a sample taken at certain time point by the initial activity of the sample.

Results obtained with detergent formulation without boric acid is shown in Figure 4. The stability of variants CM124, CM201, CM204 and CM206 was improved compared to parent Man6, when stored at high temperature 37°C for 4 weeks. At room temperature (approx. 20 - 22°C) Man6 and all the variants were equally stable. The results confirm that storage stability of the variant can be further improved with additional substitutions, such as by substitutions in positions 24 and 123.

### Example 5. Stain removal performance of mannanase variants CM201 and CM206 in full scale tests at 40°C

Stain removal performance of variants CM201 and CM206 was tested in full scale in a washing machine with liquid and powder detergents at 40°C and using water hardness of 16°dH. Parent molecule Man6 was used as reference. Tested enzyme preparations were production like samples obtained from bioreactor cultivations and they were dosed 0.5 MNU per ml of wash liquor. Activity was measured as described in Example 2. Commercial bleach detergent powder (containing all other enzymes except mannanase) was dosed 58.9 per wash (approx. 3.8 g/l) and commercial liquid detergent concentrate, containing protease and other enzymes except mannanase, was dosed 56.9 g per wash. Artificially soiled mannanase sensitive test cloths (5 items) and a natural stain (1 item) supplied from and Center for testmaterial B.V. (CFT, the Netherlands) were used as test material (Table 3). Stain swatches were stitched to huckaback towels. Artificial test cloths were first cut to pieces of 10 cm x10 cm. In addition to test monitors, 4 pieces of ballast soil sheets (WFK -SBL2004, CFT) were added in each wash. Double amount of each test monitors (tracers) were used per wash and tests were repeated. Before the main wash, ballast load (without huckaback towels containing stains) was first prewetted using an express- program. Main washing step was carried out at 40 °C using a short program (45 min) for cotton. Hardness of washing water was adjusted to 16°dH by adding suitable amounts of solutions CaCl₂ x 2 H₂O and MgSO₄ x 7 H₂O. After washing the swatches were dried overnight at indoor air, on a grid, protected against day light. The process conditions and parameters are described in Table 4.

The stain removal effect was evaluated by measuring the colour as reflectance values as described in Example 3, except final results (total stain removal effect) were shown as sum of ΔL* of each 6 stains. Color values of each stains were average of 4 swatches obtained from two washes.

Results obtained with commercial liquid detergent concentrate is shown in Figure 5 and commercial bleach detergent powder in Figure 6. Variant CM201 had better stain removal effect compared to Man6 in both detergents. Variant CM206 showed at least as good performance in the liquid and bleach detergents, when compared to parent Man6.

**Table 3. Stains used in full scale tests**

| **CFT code** | **Substrate** |
|---|---|
| **Artificial stains** | |
| C-S-06 | Salad dressing with natural black on cotton |
| C-S-05S | Mayonnaise, with carbon black on cotton |
| E-165 | Chocolate pudding mannanase sensitive on cotton |
| C-S-73 | Locust bean gum, with pigment on cotton |
| C-S-43 | Guar gum, with carbon black on cotton |

| **Natural stain** | |
|---|---|
| WEL-067kc | Chocolate ice cream, own label on knitted cotton, Ø = 5cm |

**Table 4. Process parameters and conditions used in full scale tests**

| | |
|---|---|
| Machine | Miele Softronic W1935 |
| Program | Pre-wetting: Express 20 (40°C, 20 min) |
| | Main wash: 40°C Short program (Cotton), approx. 45 min |
| Hardness of water (main wash) | about 16°dH |
| Ballast load | 3.1 kg clean, white ballast cloth (terry towels, tea towels, vests, covers, huckaback towels) |
| Detergent dosage | Liquid detergent 56.9 g or 58.9 g bleach detergent powder. |
| Amount of tracers | 2 of each type/wash |
| Number of tests | 2 |

### Example 6. Stability of variants CM201 and CM206 in liquid detergents compared to commercial mannanase

A production like sample was obtained from bioreactor cultivation of CM201 and CM206 was tested for stability in liquid detergents at various temperatures for several weeks, using a preparation of commercial mannanase for comparison.

Tests were carried out in commercial liquid detergent concentrate without boric acid (same as used in Example 4) and in another commercial liquid detergent concentrate containing boric acid. Detergents contained protease and other enzymes but no mannanase. Mannanases were added 4 w/w-% in detergent. Samples were incubated in plastic tubes with caps at 37°C for 4 weeks and at 30°C and room temperature (20 - 22°C) almost 7 or 8 weeks. The activity was measured at certain intervals with the activity assay described in Example 2 except using 60 min incubation time. Results were calculated as residual activity (%), which was obtained by dividing the activity of a sample taken at certain time point by initial activity of the sample.

Variants CM201 and CM206 had considerably better stability compared to a commercial mannanase both in the liquid detergent with boric acid (Figure 7a) and in the liquid detergent without boric acid (Figure 7b).

### Example 7. Performance of variants CM201 and CM206 compared to commercial mannanase in small scale tests at 40 °C in Launder-Ometer

Enzyme preparations of CM201 and CM206 obtained from bioreactor cultivations were tested in commercial bleach detergent powder at 40°C and water hardness of 16°dH, as described in Example 3, using a commercial mannanase as reference. Enzymes were dosed as activity units (MNU) per ml of wash liquor, representing a typical dosing range of of commercial mannanases also if calculated as % enzyme product of detergent weight).

Results are shown in Figure 8. Variants CM201 and CM206 had considerably better stain removal performance compared to the commercial mannanase in bleach containing detergent.

### REFERENCES

Joutsjoki VV, TK Torkkeli and KMH Nevalainen. 1993. Transformation of Trichoderma reesei with the Hormoconis resinae glucoamylase P (gamP) gene: production of a heterologous glucoamylase by Trichoderma reesei. Curr. Genet. 24:223-228.
Karhunen T, A Mäntylä, KMH Nevalainen and PL Suominen. 1993. High frequency one-step gene replacement in Trichoderma reesei. I. Endoglucanase I overproduction. Mol. Gen. Genet. 241:515-522.
Paloheimo M, A Mäntylä, J Kallio, and P Suominen. 2003. High-yield production of a bacterial xylanase in the filamentous fungus Trichoderma reesei requires a carrier polypeptide with an intact domain structure. Appl. Env. Microbiol. 69:7073-7082.
Penttilä M, H Nevalainen, M Rättö, E Salminen and J Knowles. 1987. A versatile transformation system for the cellulolytic filamentous fungus Trichoderma reesei. Gene 61:155-164.
Sambrook J and DW Russell. 2001. Molecular cloning, a laboratory manual. Cold Spring Harbor Laboratory, New York, US.

## Claims

1. A variant of mannanase, wherein the variant has:
at least 90%, but less than 100%, sequence identity with SEQ ID NO: 1,
a substitution of an amino acid in position 256, and
mannanase activity.

2. The variant of claim 1 wherein at least position S256 of SEQ ID NO: 1 is substituted.

3. The variant of claim 1 or 2 comprising at least the following substitutions:
positions 24 and 256;
positions 24, 123 and 256; or
positions 123 and 256.

4. The variant of any one of claims 1-3 comprising at least the following substitutions:
positions V24I and S256A; or
positions V24I and S256G; or
positions V24I, V123I, S256A; or
positions V24I, V123I, and S256G; or
positions V123I and S256A; or
positions V123I and S256G.

5. An enzyme composition comprising the variant of any one of claims 1-4 and:
a. at least one stabilizer selected from polyol, propylene glycol, polyethylene glycol, hexylene glycol, glycerol, a sugar, sugar alcohol, polysaccharide, lactic acid, peptide, surfactant, or a combination thereof; or at least one preservative or buffering agent selected from organic acid, citric acid, ascorbic acid, benzoic acid and their salts and derivatives, sodium benzoate, benzoate, hydroxybenzoate and derivatives, phosphate, sorbic acid, sodium sorbate, sorbate, salts, sodium chloride or potassium chloride, 1,2-Benzisothiazolin-3-one (BIT) or a combination thereof;
b. optionally at least one inhibitor selected from boric acid, boric acid derivative, aromatic borate ester, 4-formylphenyl boronic acid, phenyl boronic acid derivative, a peptide compound with inhibitorial function, or a combination thereof;
c. optionally at least one enzyme selected from protease, amylase, cellulase, lipase, xylanase, mannanase, cutinase, esterase, phytase, nuclease, pectinase, pectinolytic enzyme, pectate lyase, carbohydrase, arabinase, galactanase, xanthanase, xyloglucanase, laccase, peroxidase and oxidase with or without a mediator, or a combination thereof; and
d. optionally at least one filler selected from maltodextrin, flour, sodium chloride, sulfate, sodium sulfate, or a combination thereof.

6. The enzyme composition of claim 5 in the form of a liquid composition or a solid composition, solution, dispersion, paste, powder, granule, granulate, coated granulate, tablet, cake, crystal, crystal slurry, gel, or pellet.

7. A detergent composition comprising the variant of any one of claims 1-4 or the enzyme composition of claim 5 or 6.

8. The detergent composition of claim 7 in the form of a liquid detergent or a solid detergent, a bar, a homogenous tablet, a tablet having two or more layers, a pouch having one or more compartments, a regular or compact powder, a granule, a granulate, a paste, a gel, or a regular, compact or concentrated liquid.

9. The detergent composition of claim 7 or 8 further comprising one or more additional enzyme selected from protease, lipase, cutinase, amylase, carbohydrase, cellulase, pectinase, pectatelyase, pectinolytic enzyme, esterase, mannanase, arabinase, galactanase, xylanase, oxidase, xanthanase, xyloglucanase, laccase, nuclease and/or peroxidase, preferably selected from the group consisting of proteases, amylases, cellulases and lipases.

10. A recombinant host cell comprising genetic elements that allow producing at least one recombinant polypeptide comprising the variant of any one of claims 1-4.

11. A method for producing the variant of any one of claims 1-4, comprising cultivating the recombinant host cell of claim 10, wherein
the genetic elements comprise at least one control sequence which controls the production of the recombinant polypeptide in the recombinant host cell;
the genetic elements optionally comprise at least one sequence encoding a signal sequence for transporting the recombinant polypeptide outside the host cell; and
cultivating is carried out in conditions allowing production of the recombinant polypeptide.

12. A method for degrading or modifying mannan containing material comprising treating said mannan containing material with an effective amount of the enzyme composition of claim 5 or 6 or the variant of any one of claims 1-4.

13. An animal feed comprising the enzyme composition of claim 5 or 6, or the variant of any one of claims 1-4, and at least one protein source of plant origin or a mannan containing product or by-product, and:
a. Optionally at least one enzyme selected from protease, amylase, phytase, xylanase, endoglucanase, beta-glucanase, or a combination thereof; and
b. Optionally at least one filler selected from maltodextrin, flour, salt, sodium chloride, sulfate, sodium sulfate, or a combination thereof.

14. A feed supplement comprising the enzyme composition of claim 5 or 6, or the variant of any one of claims 1-4; and
a. Optionally at least one enzyme selected from protease, amylase, phytase, xylanase, endoglucanase, beta-glucanase, or a combination thereof; and
b. Optionally at least one filler selected from maltodextrin, flour, salt, sodium chloride, sulfate, sodium sulfate or a combination thereof.

15. A use of the variant of any one of claims 1-4, or the enzyme composition of claims 5-6, in oil drilling or hydro-fracturing; in processing of coffee extract, fruit juice, pineapple juice, or soya milk; in detergent; in degrading mannan containing stains; or in degrading mannan in an aqueous solution.


SEQUENCE LISTING

<110>  AB Enzymes Oy

<120>  Mannanase variant

<130>  31894EP-1f - ALLVAR-6-EP

<160>  2

<170>  PatentIn version 3.5

<210>  1
<211>  289
<212>  PRT
<213>  Bacillus clausii

<400>  1

Gln Asn Gly Phe His Val Ser Gly Thr Glu Leu Leu Asp Lys Asn Gly
1               5                   10                  15

Asp Pro Tyr Val Met Arg Gly Val Asn His Gly His Ser Trp Phe Lys
            20                  25                  30

Gln Asp Leu Glu Glu Ala Ile Pro Ala Ile Ala Glu Thr Gly Ala Asn
        35                  40                  45

Thr Val Arg Met Val Leu Ser Asn Gly Gln Gln Trp Glu Lys Asp Asp
    50                  55                  60

Ala Ser Glu Leu Ala Arg Val Leu Ala Ala Thr Glu Thr Tyr Gly Leu
65                  70                  75                  80

Thr Thr Val Leu Glu Val His Asp Ala Thr Gly Ser Asp Asp Pro Ala
                85                  90                  95

Asp Leu Glu Lys Ala Val Asp Tyr Trp Ile Glu Met Ala Asp Val Leu
            100                 105                 110

Lys Gly Thr Glu Asp Arg Val Ile Ile Asn Val Ala Asn Glu Trp Tyr
        115                 120                 125

Gly Ser Trp Arg Ser Asp Val Trp Ala Glu Ala Tyr Ala Gln Ala Ile
    130                 135                 140

Pro Arg Leu Arg Ser Ala Gly Leu Ser His Thr Leu Met Val Asp Ala
145                 150                 155                 160

Ala Gly Trp Gly Gln Tyr Pro Ala Ser Ile His Glu Arg Gly Ala Asp
                165                 170                 175

Val Phe Ala Ser Asp Pro Leu Lys Asn Thr Met Phe Ser Ile His Met
        180                 185                 190

Tyr Glu Tyr Ala Gly Ala Asp Arg Ala Thr Ile Ala Tyr Asn Ile Asp
    195                 200                 205

Arg Val Leu Ala Glu Asn Leu Ala Val Val Ile Gly Glu Phe Gly His
210                 215                 220

Arg His His Asp Gly Asp Val Asp Glu Asp Ala Ile Leu Ala Tyr Thr
225                 230                 235                 240

Ala Glu Arg Gln Val Gly Trp Leu Ala Trp Ser Trp Tyr Gly Asn Ser
                245                 250                 255

Gly Gly Val Glu Tyr Leu Asp Leu Ala Glu Gly Pro Ser Gly Pro Leu
            260                 265                 270

Thr Ser Trp Gly Lys Arg Ile Val Tyr Gly Glu Asn Gly Leu Lys Ser
        275                 280                 285

Asp

<210>  2
<211>  870
<212>  DNA
<213>  Bacillus clausii

<400>  2
cagaacggct tccacgtctc cggcacggag ctcctggaca agaacggcga cccttacgtc      60

atgcgcggcg tcaaccacgg ccacagctgg ttcaagcagg acctcgagga agccatccct     120

gctatcgctg agacgggcgc taacacggtc cgcatggtcc tgagcaacgg ccagcagtgg     180

gagaaggacg acgctagcga gctggctcgc gtcctcgctg ctacggagac gtacggcctc     240

accacggtcc tggaggtcca cgacgctacg ggctcggacg accccgccga cctcgagaag     300

gccgtcgact actggatcga gatggctgac gtcctgaagg gcaccgagga ccgcgtcatc     360

atcaacgtcg ccaacgagtg gtacggctcc tggcgcagcg acgtctgggc cgaggcctac     420

gctcaggcta tccctcgcct ccgctcggcc ggcctctccc acacgctcat ggtcgacgct     480

gccggctggg gccagtaccc tgcttccatc cacgagcgcg gcgctgacgt ctttgcttcg     540

gaccccctga agaacaccat gttctccatc cacatgtacg agtacgctgg cgctgaccgc     600

gctaccatcg cctacaacat cgaccgcgtc ctggctgaga acctggctgt cgtcatcggc     660

gagtttggcc accgccacca cgacggcgac gtcgacgagg acgctatcct ggcttacacc     720

gccgagcgcc aggtcggctg gctggcttgg tcgtggtacg gcaactcggg cggcgtcgag     780

tacctggacc tggctgaggg cccttcgggc cctctcacga gctggggcaa gcgcatcgtc 840

tacggcgaga acggcctgaa gagcgactaa 870